# EUROPEAN PATENT SPECIFICATION

(11) **EP 2 888 012 B1**
(45) Date of publication and mention of the grant of the patent: **17.06.2020**
(21) Application number: 13779381.6
(22) Date of filing: 27.08.2013
(51) Int. Cl.: A61K 8/9789, A61Q 11/00

(54) **ORAL CARE**
MUNDPFLEGE
SOINS BUCCAUX

(30) Priority: 27.08.2012 ZA 201201415
(43) Date of publication of application: 01.07.2015
(73) Proprietor: University of Pretoria, 0083 Pretoria (ZA)
(72) Inventor: HENLEY-SMITH, Cynthia Joan, 0002 Pretoria (ZA); LALL, Namrita, 0002 Pretoria (ZA); BOTHA, Francina Susanna, 0002 Pretoria (ZA); HUSSEIN, Ahmed Abdel-fattah, 8001 Cape Town (ZA)
(74) Representative: Prugneau, Philippe
(86) International application number: PCT/IB2013/058016
(87) International publication number: WO 2014/033625

(56) References cited:
- MAUD MUCHUWETI ET AL: "Total phenolic content and antioxidant activity in selected medicinal plants of Zimbabwe", INTERNATIONAL JOURNAL OF FOOD SCIENCE AND TECHNOLOGY, vol. 41, no. s1, August 2006 (2006-08), pages 33-38, XP055119144, ISSN: 0950-5423, DOI: 10.1111/j.1365-2621.2006.01258.x
- M Gundidza ET AL: "The Essential Oil from "Heteropyxis natalensis" Harv: Its Antimicrobial Activities and Phytoconstituents", Journal of the Science of Food and Agriculture, January 1993 (1993-01), pages 361-364, XP055119161, Retrieved from the Internet: URL:http://onlinelibrary.wiley.com/store/1 0.1002/jsfa.2740630315/asset/2740630315_ft p.pdf?v=1&t=hvgdq5jd&s=be2cd0a08624a12dc60 27ae3366797ec582fd45c [retrieved on 2014-05-22]
- VAN VUUREN ET AL: "Seasonal and geographical variation of Heteropyxis natalensis essential oil and the effect thereof on the antimicrobial activity", SOUTH AFRICAN JOURNAL OF BOTANY - SUID-AFRIKAANS TYDSKRIFT VIRPLANTKUNDE, FOUNDATION FOR EDUCATION, SCIENCE AND TECHNOLOGY, PRETORIA, SA, vol. 73, no. 3, 30 July 2007 (2007-07-30), pages 441-448, XP022180704, ISSN: 0254-6299, DOI: 10.1016/J.SAJB.2007.03.010
- Siyabulela S.B.N Ntuli, SNR: "ETHNOPHARMACOLOGY AND PHYTOCHEMISTRY OF SOME SELECTED MEDICINAL PLANTS IN KWAZULUNATAL", Thesis , February 2006 (2006-02), pages 1-95, XP055118470, Retrieved from the Internet: URL:http://www.turner.ukzn.ac.za/sites/def ault/files/Ntuli_Siyabulela_S_B_N_2006_0.p df [retrieved on 2014-05-16]
- Joy R Borchardt ET AL: "Antimicrobial activity of native and naturalized plants of Minnesota and Wisconsin", Journal of Medicinal Plants Research, May 2008 (2008-05), pages 98-110, XP055119481, Retrieved from the Internet: URL:http://www.academicjournals.org/articl e/article1380378099_Borchardt%20et%20al%20 %281%29.pdf [retrieved on 2014-05-22] & GAFNER STEFAN ET AL: "Antifungal and antibacterial chalcones from Myrica serrata", PLANTA MEDICA, THIEME VERLAG, DE, vol. 62, no. 1, 1 January 1996 (1996-01-01), pages 67-69, XP009158784, ISSN: 0032-0943
- Suwimol Taweechaisupapong ET AL: "Antimicrobial effects of Boesenbergia pandurata and Piper sarmentosum leaf extracts on planktonic cells and biofilm of oral pathogens", Pakistan journal of pharmaceutical sciences, April 2010 (2010-04), pages 224-231, XP055119494, Pakistan Retrieved from the Internet: URL:http://www.ncbi.nlm.nih.gov/pubmed/203 63704 [retrieved on 2014-05-22]
- Fred Joseph Benzenhafer: "The effect of Mouthwash on the Production of Biofilm of Staphylococcus aureus and Streptococcus mutans", Internet , 29 April 2012 (2012-04-29), pages 1-9, XP002724833, Retrieved from the Internet: URL:http://www.google.de/url?sa=t&rct=j&q= &esrc=s&source=web&cd=2&ved=0CDgQFjAB&url= http%3A%2F%2Fwww.honors.ufl.edu%2Fapps%2FT hesis.aspx%2FDownload%2F1584&ei=zMh9U4yFD6 PF7Ab-poGQBA&usg=AFQjCNEGxzGfUE3jqqW90LM3Z 9w6_wi9Xg&bvm=bv.67229260,d.ZGU&cad=rja [retrieved on 2014-05-22]
- Henley-Smith, Cynthia Joan: "Identification of bioactive compounds of a South African plant extract for combating potentially pathogenic oral microorganisms", Univerity of Pretoria - Thesis and Dissertations Repository , 14 August 2012 (2012-08-14), Retrieved from the Internet: URL:https://repository.up.ac.za/handle/226 3/31149 [retrieved on 2018-05-08] -& Cynthia J. Henley-Smith: "Identification of bioactive compounds of a South African plant extract for combating potentially pathogenic oral microorganisms", Univerity of Pretoria - Thesis and Dissertations Repository , 27 September 2011 (2011-09-27), Retrieved from the Internet: URL:https://repository.up.ac.za/bitstream/ handle/2263/31149/HenleySmith_Identificati on_2012.pdf?sequence=3&isAllowed=y [retrieved on 2018-05-08]

## Description

THIS INVENTION relates to oral care. In particular the invention relates to an oral care composition, the use of an extract of *Heteropyxis natalensis* for inhibiting the growth of potentially pathogenic oral microorganisms, use of a new composition, a substance or composition for use in a method of treating periodontal disease, a new use of Aurentiacin A or derivatives thereof, and a new method of evaluating the attachment of microorganisms to an enamel surface of a tooth and the effect of a composition on such attachment.

### BACKGROUND OF THE INVENTION

Biofilms (plaque) are formed from the extensive growth of microorganisms, resulting from changes in the oral bacterial ecosystem. Once a biofilm is established it may lead to the formation of dental caries (tooth decay) or even more severe periodontal diseases. Dental caries and periodontal diseases in humans have an astonishing impact on the health and welfare of communities (Samaranayake, 2002). Sick leave, due to oral infections, and the consequent cost of dental treatment results in costing billions of dollars each year (Samaranayake, 2002). In 2007, the World Health Organization (WHO) stated that 5-10% of public health expenditure was related to dental care. Tooth decay and, to a lesser extent periodontal infections, are perhaps the most expensive infections that most individuals have to contend with, during a lifetime (Loesche, 1986). Natural plant products are becoming increasingly popular treatments, even for oral health care.

One of the fastest growing sectors in the agribusiness industry is the natural plant products which led to worldwide sales of $ 23 billion in 2002 alone. In 2008, there were approximately 85,000 medicinally useful plant species; however Africa only contributed 1% to the market even though 75% of the African population still relies on traditional herbal medicine (Makunga *et al.,* 2008).

The inventor evaluated an indigenous plant from South Africa, *Heteropyxis natalensis* for bioactivity against pathogens which cause oral infections and oral diseases in humans. Identification of the bioactive principles from *Heteropyxis natalensis* has also been attempted.

The rationale of this study was to determine the antimicrobial activity of *H*. *natalensis* against four pathogenic microorganisms, *Actinomyces israelii, Streptococcus mutans, Prevotella intermedia* and *Candida albicans.* The synergistic effect of the combination of *H. natalensis* with the essential oils *Melaleuca alternifolia* (Tea tree), *Mentha piperita* (peppermint) and a concentrated green tea extract was investigated. As well as determine bacterial adhesion in the presence of *H. natalensis.* During the study flavonoids were isolated using bioassay guided fractionation.

The present invention aims to address the need for a natural product, which can prevent the colonization of potentially pathogenic oral bacteria in the mouth.

In this specification reference is made to the following documents:
Maud Muchuweti et al. 2006. Total phenolic content and antioxidant activity in selected medicinal plants of ZIMBABWE, International journal of food and science and Technology, vol. 41, n°.s1, 2006-08-01*.*
M Gundidza et al. 1993. The essential oil from "Heteropyxis natalensis" harv: "Its antimicromial activities and Phytoconstituents", Journal of the science of food and agriculture, 1993-01-01*.*
Siyabulela S.B.N. Ntuli, SNR.2006. Ethnopharmacology and phytochemistry of some selected medicinal plants in Kwazulunatal, Thesis, 2006-02-01*.*
Alviano, W.S., Alviano, D.S., Diniz, C.G., Antoniolli, A.R., Alviano, C.S., Farias, L.M., Carvalho, M.A.R., Souza, M.M.G., Bolognese, A.M. 2008. In vitro antioxidant potential of medicinal plant extracts and their activities against oral bacteria based on Brazilian folk medicine. Archives of Oral Biology, 53(6): 545-552.
Basson, A.E. 2005. Cell Culture Manual.
Braithwaite, M., van Vuuren, S.F., Viljoen, A.M. 2008. Validation of smoke inhalation therapy to treat microbial infections. Journal of Ethnopharmacology, 119: 501-506.
Cohen, M.A., Husband, M.D., Yoder, S.L., Gage, J.W., Roland, G.E. 1998. Bacterial eradication by clinafloxacin, CI-990, and ciprofloxacin employing MBC test, in-vitro time kill and in-vivo time-kill studies. Journal of Antimicrobial Chemotherapy, 41: 605-614.
Dictionary of Natural Products, version 20.1, 2011. Chapman and Hall, London.
Dominguez, X.A., Franco, R., Zamudio, A., Barradas, D.M., Watson, W.H., Zabel, V., Merijanian, A. 1980. Flavonoids from Dalea scandens var. paucifolia and Dalea thyrsiflora. Phytochemisty, 19: 1262-1263.
Du Toit, R., Volsteedt, Y., Apostolides, Z. 2001. Comparison of the antioxidant content of fruits, vegetables and teas measured as vitamin C equivalents. Toxicology, 166: 63-69.
Eloff, J.N. 1998. A sensitive and quick microplate method to determine the minimal inhibitory concentration of plant extract for bacteria. Plant Medica, 64: 711-713.
Fang, J., Paetz, C., Schneider, B. 2011. C-methylated flavonoids and dihydrochalcones from Myrica gale seeds. Biochemical Systematics and Ecology, 39(1): 68-70.
Geoghegan, F., Wong, R.W.K., Rabie, A.B.M. 2010. Inhibitory effect of quercetin on periodontal pathogens in vitro. Phytotherapy Research, 24: 817-820.
Gundidza, M., Deans, S.G., Kennedy, A.I., Mavi, S., Waterman, P.G., Gray, A.I. 1993. The essential oil from Heteropyxis natalensis Harv: Its antimicrobial activities and phytocontituents. Journal of the Science of Food and Agriculture, 63: 361-364.
Hsieh, Y-L., Fang, J-M., Cheng, Y-S. 1997. Terpenoids and flavonoids from Pseudotsuga wilsoniana. Phytochemistry, 47(5): 845-850.
Mayer, R. 1989. Flavonoids from Leptospermum scoparium. Phytochemistry, 29 (4): 1340-1342.
McFarland, J. 1907. The nephelometer: An instrument for estimating the number of bacteria in suspensions for calculating the opsonic index and for vaccines. Journal of America Medical Association, 49: 1176.
Muanda, F.N., Soulimani, R., Dicko, A. 2011. Study on biological activities and chemical composition of extracts from Desmodium adscendens leaves. Journal of Natural Products, 4: 100-107.
Muchuweti, M., Nyamukonda, L., Chagonda, L.S., Ndhlala, A.R., Mupure, C., Benhura, M. 2006. Total phenolic content and antioxidant activity in selected medicinal plants of Zimbabwe. International Journal of Food Science and Technology, 41: 33-38.
Mustafa, K.A., Kjaergaard, H.G., Perry, N.B., Weavers, R.T. 2003. Hydrogen-bonded rotamers of 2',4',6'-trihydroxy-3'-formyldihydrochalcone, an intermediate in the synthesis of a dihydrochalcone from Leptospermum recurvum. Tetrahedron, 59: 6113-6120.
Song, J.M. and Seong, B.L. 2007. Tea catechins as a potential altenative anti-infectious agent. Expert Review of Anti-infective Therapy, 5(3): 497-506.
Van Vuuren, S.F., Viljoen, A.M., 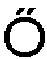 Ozek, T., Demirci, B., Ba 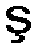 ser, K.H.C. 2007. Seasonal and geographical variation of *Heteropyxis natalensis* essential oil and the effect thereof on the antimicrobial activity. *South African Journal of Botany,* 73: 441-448.
Van Wyk, B. and Gericke, N. 2000. General medicines, Chapter 7. Dental care, Chapter 12. Perfumes and repellents, Chapter 13. In: People's plants. Briza Publications, Pretoria, South Africa, pp. 119-228.
Van Wyk, B. and van Wyk, P. 1997. Field guide to trees of Southern Africa. Struik Publishers, South Africa, pp. 198-500.
Vilela, R.M., Lands, L.C., Meehan, B., Kubow, S. 2006. Inhibition of IL-8 release from CFTR-deficient lung epithelial cells following pre-treatment with fenretinide. International Immunopharmacology, 6: 1651-1664.

### SUMMARY OF THE INVENTION

The invention relates to an oral care composition for inhibiting the growth of potentially pathogenic oral microorganisms as disclosed in claims 1 to 7 and claim 9.

The invention further relates to a substance or composition comprising the oral care composition as disclosed in claims 1 to 7, for use in a method of treating periodontal disease.

The invention further relates to a substance or composition comprising Aurentiacin A or derivatives thereof, for use in a method of treating periodontal disease.

The invention will now be described, by way of example only with reference to the following drawing(s) and table(s):

### BRIEF DESCRIPTION OF THE DRAWINGS AND TABLES

In the figure(s):
Figure 1a shows an image of an enamel surface of the extracted tooth which was exposed to stimulated saliva to form a pellicle on the enamel;
Figure 1b shows an enamel surface which was exposed to the plant extract, in accordance with an embodiment of the invention, in a carbohydrate containing growth medium (CASO), to determine if a coating similar to a normal pellicle, could be formed on the enamel (Treatment 1). This image shows the coating of the enamel surface of teeth by the plant extract;
Figure 1c shows an image of an enamel surface coated with the chemical substance, Repelcote® that prevents adhesion of bacterial cells to a surface. The enamel was exposed to a carbohydrate medium to determine if a 'pellicle' could be formed on enamel;
Figure 2 shows percentage inhibition of DPPH by Vitamin C;
Figure 3 shows percentage inhibition of DPPH by Quercetin;
Figure 4 shows percentage inhibition of DPPH by *Heteropyxis natalensis;*
Figure 5 shows percentage inhibition of DPPH by concentrated green tea extract (TEAVIGO™);
Figure 6 shows Percentage inhibition of DPPH by the synergistic compilation of *H*. *natalensis* (3.125 mg/ml), *M. alternifolia* (0.05% v/v), *M. piperita* (0.05% v/v) and concentrated green tea extract (TEAVIGO™) (2.5 mg/ml). These respective concentrations were taken as 100% initial concentration.

### DETAILED DESCRIPTION OF THE INVENTION

The invention will be more readily understood through the following detailed description and scientific study, which is not intended to limit the invention, but merely exemplify the embodiments of the invention.

### Material and methods

### Plant material

Aerial plant parts, comprising of leaves and twigs of *H. natalensis* was collected. The plant was collected from the University of Pretoria's Botanical Garden during January. A voucher specimen was prepared and identified at the H.G.W.J. Schwelcherdt Herbarium (PRU), University of Pretoria, (PRU 096405).

### Preparation of extract

The plant material was air dried at room temperature (25°C), and ground to a fine powder using a Janke & Kunkel (IKA Labortechnik, Germany) grinder. The powdered material was extracted by shaking with 400 ml ethanol (Merck Chemicals (Pty) Ltd Wadeville, South Africa) (Labcom shaker). The sample was filtered through a Whattman No.1 (110 mm diameter) (Merck Chemicals (Pty) Ltd Wadeville, South Africa) filter paper using a vacuum filter (Merck Chemicals (Pty) Ltd Wadeville, South Africa). The process was repeated several times. The solvent was evaporated in a BUCHI Rotavapor (Labotec (PTY) Ltd. Halfway House, South Africa) under reduced pressure of 40°C. The extract was further dried at room temperature after which they were subjected to antimicrobial tests.

### Antimicrobial activity

### Microbial strains

The microorganisms used in this study included *Actinomyces israelii* (ATCC 10049), *Prevotella intermedia* (ATCC 25611), *Streptococcus mutans* (ATCC 25175), *Candia albicans* (ATCC 10231) and a strain of *Candida albicans* resistant to polyenes and azoles (1051604). The bacteria were grown on Casein-peptone Soymeal-peptone Agar medium (CASO) (Merck Chemicals (Pty) Ltd Wadeville, South Africa) under anaerobic conditions in an anaerobic jar with Anaerocult® A (Merck KGaA Darmstadt, Germany), at 37 °C for 72 hours. *Actinomyces israelii* and *S*. *mutans* had CASO agar enriched with 1% sucrose (Merck Chemicals (Pty) Ltd Wadeville, South Africa). The drug susceptible and drug resistant strains of *C*. *albicans* were grown on Sabouraud Dextrose 4% Agar (SDA) (Merck Chemicals (Pty) Ltd Wadeville, South Africa), at 37 °C for 72 hours. Sub-culturing was done every second week. Inocula were prepared by suspending bacterial test organisms in their respective broths until turbidity was compatible with McFarland Standard 1 (Merck Chemicals (Pty) Ltd Wadeville, South Africa). Yeast test organisms were suspended in sterile distilled water until turbidity was compatible with McFarland Standard 1 (McFarland, 1907).

### Determination of minimum inhibitory concentration (MIC) and minimum bactericidal concentration (MBC)

The microdilution technique using 96-well micro-plates, as described by Eloff (1998) was used to obtain the MIC and MBC values of the crude extracts against the microorganisms under study. The extracts, dissolved in 10% dimethyl sulphoxide (DMSO) (Merck Chemicals (Pty) Ltd), were serially diluted in broth (enriched for *A. israelii* and *S*. *mutans*) for the bacteria and sterile water for the *Candida* species; in the 96-well plate adding 48 hour old microorganisms grown at 37°C. The final concentration of extracts ranged from 12.5-0.10 mg/ml and the positive control, 5% chlorhexidine gluconate (CHX) (Dental Warehouse, Sandton, South Africa), ranged from 12.5-3.8 x 10⁻⁴ mg/ml. Amphotericin B (Davis Diagnostics, Gauteng) an established antifungal drug, ranging from 0.2 mg/ml to 1.5 x 10⁻³ mg/ml, was included for the *Candida* assays. The highest concentration of the solvent DMSO (2.5%) was found to be non-toxic to the microorganisms tested. *Actinomyces israelii* and *S*. *mutans* were incubated at 37°C, under anaerobic conditions, for 24 hours; *P. intermedia* was incubated at 37°C, under anaerobic conditions, for 48 hours; and *C*. *albicans* was incubated at 37°C with *p-*iodonitrotetrazolium violet (INT) (Sigma-Aldrich, South Africa) already added, under moist aerobic conditions, for 24 hours.

To indicate bacterial growth, 50 µl of (0.2 mg/ml) INT, was added to micro-plate wells and incubated at 37°C, under anaerobic conditions, for 20-60 minutes until a red colour developed. The MIC was defined as the lowest concentration that inhibited the colour change of INT. The MBC was determined by adding 50 µl of the suspensions from the wells, which did not show any growth after incubation during MIC assays, to 150 µl of fresh broth. These suspensions were reincubated at 37°C for 28 hours (48 hours for *P. intermedia*), under anaerobic conditions. The MBC was determined as the lowest concentration of extract which inhibited 100% growth of microorganisms (Cohen *et al.,* 1998).

### Determination of cytotoxicity

Microtitre plates with Vero and HEp-2 cells (Highveld Biological, Gauteng) were used for testing the four best ethanol extracts for cytotoxicity following the method of Basson (2005). Cytotoxicity was measured by the XTT (Sodium 3'-[1-(phenyl amino-carbonyl)-3,4-tetrazolim]-bis-[4 methoxy-6-nitro] benzene sulfonic acid hydrate) method using the cell proliferation kit II (Roche Diagnostics GmbH). A hundred microliters of Vero and HEp-2 cells (1 x 10⁵ ml) were seeded onto micro-plates and incubated for 24 hrs to allow the cells to attach to the bottom of the plate. Dilution series were made of the extracts and the various concentrations (400-3.1 µg/ml) were added to the micro-plate and incubated for 72 hours. The XTT reagents were added to a final concentration of 0.3 mg/ml and the cells were incubated for one to two hours. The positive drug controls Doxorubicin HCI (Sigma-Aldrich, South Africa) and Actinomycin D (Sigma-Aldrich, South Africa), at concentration ranges of 0.78-0.01 µg/ml, were included in the assay. After incubation the absorbance of the colour was spectrophotometrically quantified using an enzyme-linked immunosorbent assay (ELISA) plate reader (BIO-TEK Power-Wave XS, Weltevreden Park, South Africa), which measured the optical density at 490 nm with a reference wavelength of 690 nm. The assay was carried out in triplicate.

### Synergistic assay

Aerial plant parts, comprising of leaves and twigs of *H. natalensis* was collected. The plant was collected from the University of Pretoria's experimental farm during January. A voucher specimen was prepared and identified at the H.G.W.J. Schwelcherdt Herbarium (PRU), University of Pretoria, (PRU 096405). *Melaleuca alternifolia* essential oil (Holistic Emporium cc, Gauteng, South Africa), *Mentha piperita* essential oil (Holistic Emporium cc, Gauteng, South Africa), and TEAVIGO™ (Chempure (Pty) Ltd, Silverton, South Africa), were purchased as well for the present investigation.

The *H. natalensis* plant material was air dried at room temperature (25°C), and ground to a fine powder using a standard food processor. The powdered material was extracted with ethanol (Merck Chemicals (Pty) Ltd Wadeville, South Africa) under pressure (100 bar) and regulated temperature of 50°C in a BUCHI Speed Extractor, E-916 (BUCHI Labortechnik AG, Switzerland). The solvent was evaporated on low boiling point in a Genevac, EZ-2 plus (Genevac SP Scientific, UK), after which the extract was subjected to antimicrobial tests.

The microorganisms used in this study included *Prevotella intermedia* (ATCC 25611), *Streptococcus mutans* (ATCC 25175) and *Candida albicans* (ATCC 10231). The bacteria were grown on Casein-peptone Soymeal-peptone Agar) (CASO) (Merck Chemicals (Pty) Ltd Wadeville, South Africa) enriched with 1% sucrose (Merck Chemicals (Pty) Ltd Wadeville, South Africa) under anaerobic conditions in an anaerobic jar with Anaerocult® A (Merck Chemicals (Pty) Ltd Wadeville, South Africa), at 37 °C for 48 hours. *Candida albicans* was grown on Sabouraud Dextrose 4% Agar (SDA) (Merck Chemicals (Pty) Ltd Wadeville, South Africa), at 37 °C for 48 hours. Sub-culturing was done every second week. Inocula were prepared by suspending bacterial test organisms in their respective broths until turbidity was compatible with McFarland Standard 1 (Merck Chemicals (Pty) Ltd Wadeville, South Africa) (McFarland, 1907).

To determine the effects of combinations of *H. natalensis, M. alternifolia* essential oil, *M. piperita* essential oil and TEAVIGO™, the MIC of each component was determined first using the antimicrobial microplate method of Eloff (1998). A stock solution of the ethanol extract of *H*. *natalensis* was prepared in 20% dimethyl sulphoxide (DMSO) (Merck Chemicals (Pty) Ltd); while TEAVIGO™ was dissolved in distilled water. The stock solutions were serially diluted in enriched Casein-peptone Soymeal-peptone medium broth (Merck Chemicals (Pty) Ltd) for the bacteria and Sabouraud Dextrose 4% broth (Merck Chemicals (Pty) Ltd) for *Candida;* in the 96-well plate adding a McFarland Standard 1 inoculum of 48 hour old microorganisms grown at 37°C. The final concentration of the extract and TEAVIGO™ ranged from 0.10-12.5 mg/ml and the positive control, 1.25% v/v chlorhexidine gluconate (CHX) (Dental Warehouse, Sandton, South Africa), ranged from 4.77x10⁻⁶-0.31% v/v. The essential oils were dissolved in 10% Tween (80) (Merck Chemicals (Pty) Ltd Wadeville, South Africa). The final concentration tested of the essential oils ranged from 1.6x10⁻⁵-1.25% v/v. The highest concentration of the solvent Dimethyl sulphoxide (DMSO) (5%) and Tween 80 (2%) was found to be non-toxic to the microorganisms tested. The inoculated plates were incubated at 37°C, under anaerobic and aerobic conditions respectively for 24 hours before adding the colour indicator PrestoBlue (Lall *et al.,* 2013). The minimum inhibitory concentration (MIC) was defined as the lowest concentration that inhibited the colour change of PrestoBlue.

The synergistic activity of the samples was determined using a modified checkerboard method. This process was repeated for all combinations of the 4 agents for each microorganism tested.

### Anti-adherence

### Cytokine assay

Levels of IL-8 in the supernatants were determined using an enzyme-linked immunosorbent assay (ELISA) kit (Pharmigen, OptEIA Human IL-8 Set, catalog no. 555244) obtained from BD Bioscience. Cells were grown in pre-coated T-75 flasks in Eagle's minimum essential medium (MEM) containing 10% fetal bovine serum (FBS) and re-fed every 2-3 days until confluent. The confluent, adherent monolayers were then released from the plastic surface after treatment with polyvinyl-pirrolidone (PVP)-trypsin-EDTA and were seeded to 24-well plates for 24 hrs before receiving the treatments. The cells were rinsed three times with phosphate-buffered saline (PBS) buffer and 1 ml antibiotic free medium was added to the cells which were then treated with *H. natalensis* at non-cytotoxic concentrations established previously in cell culture studies.

To determine if the plant extract had an effect on IL-8 release from oral epithelial cells, extracts were added to the cells at concentrations varying from 12.5-200 µg/ml. Time-dependent studies were carried out. Plant extracts were added to HEp-2 cells and one hour later *A. israelii* was added. Plant extracts and *A. israelii* were added to HEp-2 cells together and lastly *A. israelii* was added to HEp-2 cells with the plant extracts being added one hour later. *Heteropyxis natalensis* extracts were added in duplicate to each well. A negative control of only *A. israelii* and HEp-2 cells was included. The plates were incubated overnight at 37°C in 5% CO₂.

After the treatment described above, the supernatants were collected to determine IL-8 released using a commercially available ELISA kit. Briefly, 96-well plates were coated with 100 µl of capture antibody (anti-human IL-8 monoclonal antibody) incubated overnight, washed three times with 0.05% Tween-20 in FBS and coated with PBS with 10% FBS in order to block non-specific binding . Known concentrations of IL-8 (standard) and the samples containing the IL-8 released by the cells after treatment (supernatant) were added as aliquots into appropriate wells, incubated for two hours and decanted from the wells. Aspirated and washed five times. Biotinylated (the attachment of a biotin residue to a biological macromolecule in order to label it) anti-human IL-8 monoclonal antibody and streptavidin-horseradish peroxidase conjugate were added and incubated for one hour. After washing the plate, a solution containing a substrate for the enzyme (3,3',5,5' tetramethylbenzidine-peroxide chromogen) present in the anti-IL-8 and enzyme reagent mixture was added and the plate was incubated for 30 minutes. The reaction was stopped using a 2N sulfuric acid (H₂SO₄) solution and the absorbance was read at 450 nm using an ELISA plate reader (BIO-TEK Power-Wave XS, Weltevreden Park, South Africa). The absorbencies were then used to calculate the IL-8 concentration from the standard curve (Vilela *et al.,* 2006).

### Ultrastructure

To determine whether or not the plant extract affected the attachment of *S*. *mutans* to the enamel of human teeth, time-dependent studies were carried out. Firstly *H*. *natalensis* extract, at an MIC concentration of 1.82 mg/ml and sub-MIC value of 0.91 mg/ml, were added to the prepared enamel fragments and one hour later *S*. *mutans* was added. Secondly plant extracts and *S*. *mutans* were added to the enamel fragments together and lastly *S*. *mutans* was added to the enamel fragments with the plant extracts being added one hour later. The plant extract was added in duplicate to each well. The positive control consisted of enamel fragments being coated with a chemical that prevents adhesion of bacteria. A negative control of only *S*. *mutans* and enamel fragments was included. The plates were incubated overnight.

Teeth collected for this study had been extracted from human patients for reasons other than the purpose of this study. Each patient who attended the extraction clinic of the University of Pretoria must complete and sign a patient information leaflet and informed consent form. Non-carious, recently extracted human teeth, were collected from Dental Clinics. Only teeth which had been extracted for periodontal or orthodontic reasons were used. Ethical and safety guidelines for the handling of human teeth and laboratory research were strictly followed. Immediately after extraction the teeth were rinsed in running water. Thereafter the teeth were placed in distilled water in an ultrasonic water bath and sonicated for periods of fifteen minutes in clean distilled water until all loose biological material was removed and stored at 4 °C. The crowns of the teeth were removed by horizontally sectioning at the cementalenamel junction with a diamond wafering blade in an Isomet 11-1180 low speed saw (Buehler Ltd., Lake Bluff, Illinois, USA) under permanent water irrigation. The crowns were further cut into blocks; the samples were placed in sterile Ringer's solution (Merck SA (Pty) Ltd., Halfway House, South Africa) and sterilized at 125 °C for 15 min.

The rest of the procedure was carried out under sterile conditions. Sterility was maintained for the duration of the entire experiment that was conducted in a positive sterile airflow laboratory, using sterile instruments as well as gloves and masks. Before sterilisation some of the enamel blocks used as samples were cleaned, dried and coated with 2% Dimethyl dichlorosilane in 1,1,1-trichloroethane (Repelcote® - Saarchem-Holpro Analytic (Pty.) Ltd., 40 Fransen Street, Chamdor, Krugersdorp) in order to create a repellent surface that would prevent organisms attachment, utlized as a positive control.

All samples were placed in 24 well tissue culture plates containing 1 ml CASO broth and incubated at 37°C for 1 hour. For Treatment 1, 1ml of the different plant extracts were added and incubated at 37°C for 1 hour, after which 1% MacFarland Standard-1 bacterial suspension was added. For Treatment 3, the bacterial suspension was added to the enamel blocks and incubated for an hour, after which the different plant extracts were added. For Treatment 2, the different plant extracts and the 1% MacFarland Standard-1 bacterial suspension were added at the same time, the plates incubated anaerobically as described in a shake incubator for 24 and 48 hours. One enamel block with the organism tested was used as a positive control and all the samples were prepared for the Scanning Electron Microscope (SEM) as the positive control.

### Preparation for Scanning Electronmicroscopy (SEM)

One sample was collected from each of the tissue culture wells containing the different plant extract concentrations at 24 and 48 hrs for SEM to determine the colonization of the organisms on enamel. The samples were prepared according to standard methods for biological SEM evaluation according to Glauert (1975) and Hayat (1981).

### Purification of active compounds

The dried ethanolic extract (60 g) was subjected to fractionation on a silica column (10 x 70 cm) using a gradient of hexane:ethyl acetate of increasing polarity (0% to 100% ethyl acetate) as eluent. Twenty-nine fractions were collected and those with similar thin layer chromatography (TLC) profiles were combined together (TLC plates were developed using hexane:ethyl acetate (7:3); hexane:ethyl acetate (8:2); dichloromethane:methane (99.5:0.5) and dichloromethane:methane (99:1) as eluent. Acidic vanillin; 0.34% vanillin in 3.5% sulphuric acid in methanol; was used for detection). Thirteen major fractions (1B to 13B) were obtained and tested for antibacterial activity against *A. Israelii* (Table 1).

**Table 1: Average minimum Inhibitory concentration (MIC) and minimum bactericidal concentration (MBC) of the Fractions**

| Fraction | MIC (mg/ml) | MBC (mg/ml) |
|---|---|---|
| 1B | >12.5 | Na*^{a}* |
| 2B | >12.5 | Na |
| 3B | >12.5 | Na |
| 4B | >12.5 | Na |
| 5B | 9.375 | 9.375 |
| 6B | 9.375 | 9.375 |
| 7B | 0.830078 | 1.611328 |
| 8B | 0.830078 | 2.34375 |
| 9B | 1.5625 | 2.34375 |
| 10B | 2.213542 | 8.203125 |
| 11B | 4.25 | 12.5 |
| 12B | 12.5 | Na |
| 13B | 1.822917 | 5.46875 |
| Positive control*^{b}* | 0.018311 | 0.048828 |

| | | |
|---|---|---|
| ^{a}Na: Not active; ^{b} Chlorhexidine | | |

Based on the antibacterial results and preliminary TLC plates; Fractions 7B (2 g), 9B (3 g), 11B (0.8 mg) and 12B (1 g) were chromatographed separately using Sephadex columns (Sigma-Aldrich, South Africa). Fraction 7B was chromatographed using dichloromethane:methane (99.5:0.5). Sixty-four subfractions were collected, spotted on TLC plates and developed in dichloromethane:methane (99:1). Pure compound 1 was obtained. Fraction 9B was chromatographed using 100% ethanol. Ninety-one subfractions were collected, spotted on TLC plates and developed in dichloromethane:methane (99.5:0.5). Pure compounds 2 and 3 were obtained. Fraction 11B was chromatographed using dichloromethane:methane (99:1). Fifty-five subfractions were collected, spotted on TLC plates and developed in dichloromethane:methane (99:1). Pure compound 4 was obtained. Fraction 12B was chromatographed using dichloromethane:methane (99:1). Seventy-one subfractions were collected, spotted on TLC plates and developed in dichloromethane:methane (99:1). Pure compound 5 was obtained.

### Antioxidant Assay

Evidence suggests an association between periodontal diseases and an imbalance between oxidants and antioxidants due to both an increase in free radical production and a decrease in the antioxidant activity of saliva. Reactive oxygen species (ROS) have been linked to the destruction of periodontal tissues (Alviano *et al.,* 2008).

DPPH (2,2-diphenyl-1-picrylhydrazyl hydrate) is a stable free radical with an unpaired valence electron at a nitrogen atom forming a bridge. The free radical can initiate a chain reaction which causes the removal of an electron from another molecule to complete its own orbital. This free valence electron forms the basis of the DPPH assay as samples are tested to determine the scavenging capability of this DPPH radical.

A modified antioxidant assay was used (du Toit *et al.,* 2001). A 2% stock solution of *M*. *piperita* and *M*. *alternifolia,* a 2 mg/ml stock solution of ascorbic acid (vitamin C) (Sigma-Aldrich (Pty) Ltd, Aston Manor, South Africa) and *H. natalensis,* a 1 mg/ml stock solution of Quercetin, a 500 µg/ml stock solution of TEAVIGO™ and a synergistic compilation of *H. natalensis* (3.125 mg/ml), *M. alternifolia* (0.05% v/v), *M. piperita* (0.05% v/v) and TEAVIGO™ (2.5 mg/ml) were prepared.

In a 96 well ELISA plate, 20 µl of each sample was added to 200 µl dH₂O (except for Quercetin which was added to 200 µl EtOH), serial dilutions were done. After which 90 µg/ml DPPH (Sigma-Aldrich (Pty) Ltd, Aston Manor, South Africa) dissolved in ethanol (40 µg/ml) were added to all wells. All samples were prepared in triplicate. Vitamin C was used as a positive control, an extract control and blank controls were also done. The absorbance was measured in an ELISA plate reader at 515 nm (du Toit *et al.,* 2001).

### Results and discussion

The MIC exhibited by the ethanol extract of *H. natalensis* was found to be 1.82 mg/ml and 0.88 mg/ml against S. *mutans* and *A. israelii* respectively. The plant exhibits moderate toxicity against HEp-2 cells with a 50% inhibition (IC₅₀) of 33.66 ± 0.04 µg/ml (Table 2)

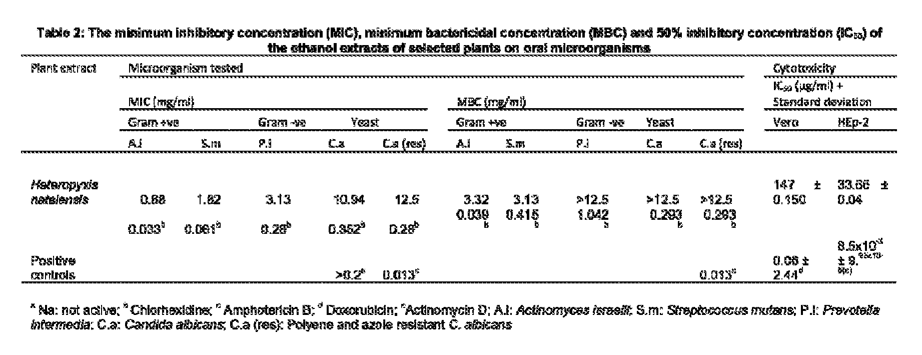

### Synergistic assay

The modified checkerboard method was utilized for the reduction of MIC values. This method provided numerous concentration variables for the agents under investigation and their inhibition potential.

**Table 3: Comparison of the minimum inhibitory concentrations of the tested agents alone and in combination after utilizing statistical analyses**

| | ***P. intermedia*** | | ***C. albicans*** | | ***S. mutans*** | |
|---|---|---|---|---|---|---|
| | **Agent alone** | **Combination** | **Agent alone** | **Combination** | **Agent alone** | **Combination** |
| ***H. natalensis* (mg/ml)** | 12.50 | 3.13 | 8.33 | 3.13 | 2.60 | 0.78 |
| **TEAVIGO™ (mg/ml)** | >12.5 | 2.00 | 10.42 | 4.00 | 1.30 | 0.78 |
| ***M. piperita* (% v/v)** | 0.20 | 0.05 | 0.10 | 0.05 | 0.10 | 2 x 10⁻³ |
| ***M. alternifolia* (%v/v** | 0.29 | 0.05 | 0.24 | 0.01 | 0.29 | 4 x 10⁻⁴ |

There is a reduction in the MIC values of each agent alone, when used in combination for each of the microorganisms tested (Table 3). And therefore we can state that there is an overall increase in the inhibitory activity when the agents are used in combination.

### Cytokine assay

Although the plants themselves did not induce a release of IL-8 (not shown on graph) there seems to be a negative interaction between *H. natalensis* and *A. israelii* that induces the release of IL-8, as all the IL-8 readings are higher than that of the negative control of the bacteria and cells only, at 4.6 pg/ml. Although *H. natalensis* inhibited *A. israelii* growth at 0.88 mg/ml and is bacteriostatic at 3.32 (mg/ml); it does not appear to hinder the adhesion mechanism of *A. israelii* and must therefore affect the microorganism in another way.

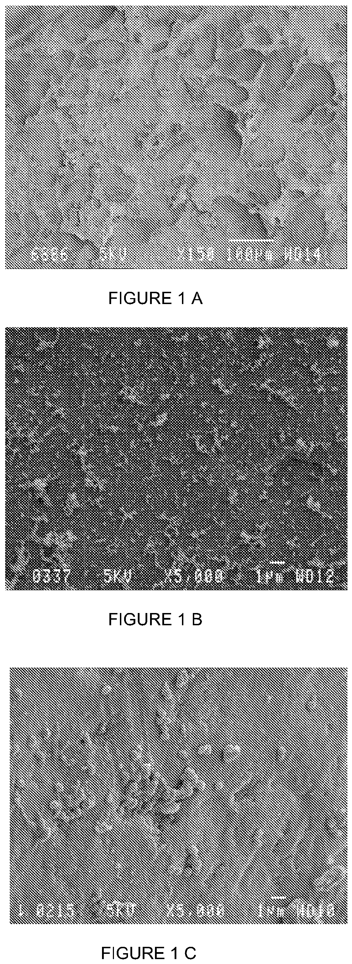

Figure 1a, b and c represents the comparison of the pellicle as formed on the enamel surface of teeth.

### Identification of the isolated compounds

The identification of the compounds was done by ¹H and ¹³C nuclear magnetic resonance (NMR) and distortionless enhancement by polarization (DEPT). The MIC, MBC and IC₅₀ of the isolated compounds against *Actinomyces israelii* and on HEp-2 cells are shown in Table 4.

**Table 4: The minimum inhibitory concentration (MIC), minimum bactericidal concentrations (MBC) and 50% inhibition of cell growth (IC₅₀) of the isolated compounds against Actinomyces israelii and on HEp-2 cells**

| Isolated compounds | MIC(mg/ml) | MBC (mg/ml) | IC₅₀ (µg/ml) + S.D. |
|---|---|---|---|
| *Heteropyxis natalensis* | 1.5625 | 1.5625 | 33.66 ± 0.04 |
| 5-hydroxy-7-methoxy-methylflavanone (**3**) | - | - | - |
| Aurentiacin A (**1**) | 0.0625 | 0.0625 | 6.36 ± 5.095 |
| Cardamomin (**2**) | >1 | NA*^{a}* | 52.12 ± 9.41 |
| 3,5,7-trihydroxyflavan (**5**) | >1 | NA | >200 |
| Quercetin (**4**) | 1 | 1 | 186.7 ± 42.605 |
| Positive control | 0.024*^{b}* | 0.024*^{b}* | 9.6x10⁻³ ± 0.0003*^{c}* |

| | | | |
|---|---|---|---|
| ^{a}NA: Not active; ^{b}Chlorhexidine; ^{c}Actinomycin D | | | |

### Fraction 9B subfraction 2-5, sub-subfraction 4

Compounds 2 and 3 were recognised as chalcones with different substitution patterns in ring B, while ring C is not substituted. Compound 3 was identified based on the spectral data of ¹H and ¹³C NMR. It showed in ¹H NMR signals of an unsubstituted ring C at 7.72 (2H 2, 6), 7.45 (3H, 3, 4, 5). It also showed signals of trans alkene at 8.05 and 7.73 (1H, d each, J=15.4 Hz) and two aromatic protons at 6.08, 6.02 (s each, H3' and 5') in addition to a methoxyl signal at 3.97.

The ¹³C NMR data showed 16 carbon signals including a carbonyl group at 193.0 ppm. DEPT-135 showed 10 protonated carbons one of them is the methoxyl group at δ_{C} 56.4 and two α,β double bonds adjacent to the carbonyl group at 142.6 and 128.4; while the other signals were attributed to the aromatic carbons of both ring B and ring C.

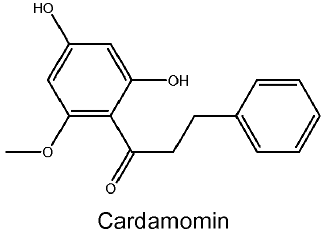

The data given established the structure of Cardamomin, or cardamonin, as compound 2. Cardamomin is a 2'- Me ether derivative of 2',4',6'-Trihydroxychalcone and has been previously isolated from *Alpinia katsumadai, Boesenbergia pandurata, Comptonia peregrina, Myrica pensylvanica, Piper* sp., *Populus* sp. and *Dracaena draco.* Cardamomin has been shown to inhibit pro-inflammatory mediators and therefore has anti-inflammatory activity and has also shown antitumor activity (Dictionary of Natural Products, 2011).

### Fraction 7B subfraction 26 sub-subfraction 30

¹H NMR showed downfield signals at δ_{H} 13.51 ppm of a chelated hydroxyl at C-6'; signals of monosubstituted benzene ring at 7.40 (*s*, 3H), 7.67 (*s*, 2H) and another singlet signal of an aromatic proton at 6.31, two doublets at 8.02, 7.79 (d, J=15.8 Hz), a low field methyl group at 2.09 and a methoxyl group at 3.67.

The ¹³C NMR data showed 17 signals; two methyls (60.9,7.2), eight methines at 127.1 (C-2,6), 128.3 (C-3,5), 125.9 (C-4), 142.0 (C-7), 129.6 (C-8) and 98.6 (C-3) and a carbonyl group at 191.9 (C-9) in addition to seven quarternary carbons.

The above data indicated the presence of a chalcone derivative with no oxygenation at ring B. Ring A showed only one signal of an aromatic proton (δ_{H} 6.31) which showed correlation heteronuclear multiple bond correlation (HMBC) to C-1' (107.3) and C-3' (110.1). The hydroxyl proton (δ_{H} 13.51) showed correlation with C-1' and C-5' H-5' / C6', C1', C3' and C4'. This relation could establish the substitution pattern of ring A to 2'-methoxy,4',6' dihydroxy. The compound (1) was identified as 2'-Me ether derivative of 2',4',6'-trihydroxy-3'-methylchalcone; or more commonly known as aurentiacin A.

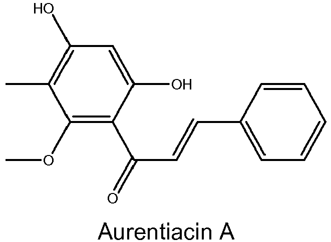

Aurentiacin A has previously been isolated from *Didymocarpus aurentiacum, Comptonia peregrina, Dalea* species including *Dalea scandens* var. *paucifolia* (medicinal value in Mexico), *Myrica pensylvanica* and also from *Dracaena* species (Dictionary of Natural Products, 2011). Aurentiacin A, isolated from *Myrica serrate,* inhibits the growth of *Cladosprium cucumerinum, Bacillus subtilis* and *E. coli* (Dominguez *et al.,* 1980; Gafner *et al.,* 1996).

### Fraction 9B subfraction 2-5, sub-subfraction 2

The compound showed to be C-flavonoid from its spectroscopic data. The ¹H NMR showed signals 5.44 (d, J=14.2 Hz) of H2. Two H-3 protons appeared at 2.64 (d, J=16.6 Hz) and 3.04 (dd, 16.6, 14.2 Hz). The signal at 6.35 (s) was attributed to H-8 and those at 7.40 and 7.53 attributed to monosubstituted ring B, methyl (attached at C-6) appears at 2.04 and a methoxyl group at 3.76.¹³C NMR and DEPT-135 showed a two methyl signals at 61.0 and 8.0, a methylene group at 45.9 and 5 methine signals at 79.3 (C2), 100.0 (C8), 126.6, 129.1 and 129.2 of ring B. Other signals for quaternary carbons belong to C-4 (188.0), C-7(163.4), C-5(162.9), C-9(116.5), C1'(140.4) and C-6 (113.7).

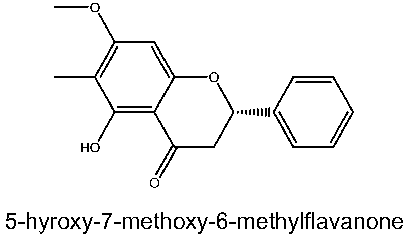

The above mentioned data established the structure of the compound (3) as 5-hyroxy-7-methoxy-6-methylflavanone. Previously isolated from *Leptospermum scoparium* (used in Australian and New Zealand traditional medicine), *Leptospermum recurvum, Piper carniconnectivum, Pseudotsuga wilsoniana* (used as building wood in Taiwan), the seeds of *Myrica gale* (fruit is a beer additive, essential oil is an insect repellent) and a trace constitute of *Pityrogramma triangularis* (Dictionary of Natural Products, 2011; Facundo & Braz-Filho, 2004; Fang *et al.,* 2011; Hsieh *et al.,* 1997; Mayer, 1989; Mustafa *et al.,* 2003).

### Fraction 12B subfraction 56, sub-subfraction 70

The ¹H NMR showed five aromatic signals at 6.80 (m), two meta coupled protons at 5.92, 5.84 (br. *s* each), two protons germinal to hydroxyl groups at 4.55 (d, J=7.0 Hz), 3.36 (m) [C-2, C-3], in addition to methylene protons at 2.84 (dd, J=16.1, 4.8 Hz) and 2.49 (dd, J=16.1, 8.4 Hz).

This compound has two possibilities, depending on the alpha D value.

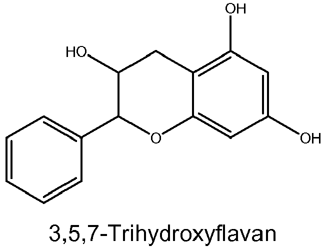

The above ¹H NMR data established the structure of compound 5 as 3,5,7-Trihydroxyflavan. There are two variants of the compound, namely the 2*R*, 3*R* and 2*R*, 3*S* forms. The 2*R*, 3*R* form is known as distenin, previously isolated from *Dennstaedtia distenta*; while the 2R, 3S form is known as 3-Oxykoaburagenin, previously isolated from the leaves of *Enkianthus nudipes* (Dictionary of Natural Products, 2011).

### Fraction 11 subfraction 10

This compound was identified as the well-known flavonoid, quercetin. The ¹H NMR showed a typical quercetin signal at 7.82 (br. *s*, H-2'), 7.70 (br. d, J=8.0 Hz, H-6'), 6.99 (br. d, J=8.0 Hz, H-5'), 6.52 (br. *s*, H-8) and 6.26 (br. *s*, H-6).

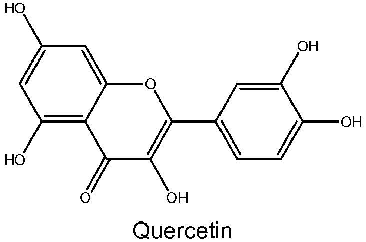

The above mentioned data established the structure of the compound (4) as 3,3',4',5,7-pentahydroxyflavone, or more commonly known as quercetin. This compound has been previously isolated from many plant species, especially fruits, such as *Helichrysum, Euphorbia* and *Karwinskia* spp. The compound is present in almost all species of the Umbelliferae family and is present in the Solanaceae, Rhamnaceae and Passifloraceae families (Dictionary of Natural Products, 2011; Geoghegan *et al.,* 2010; Muanda *et al.,* 2011).

### Antioxidant Assay

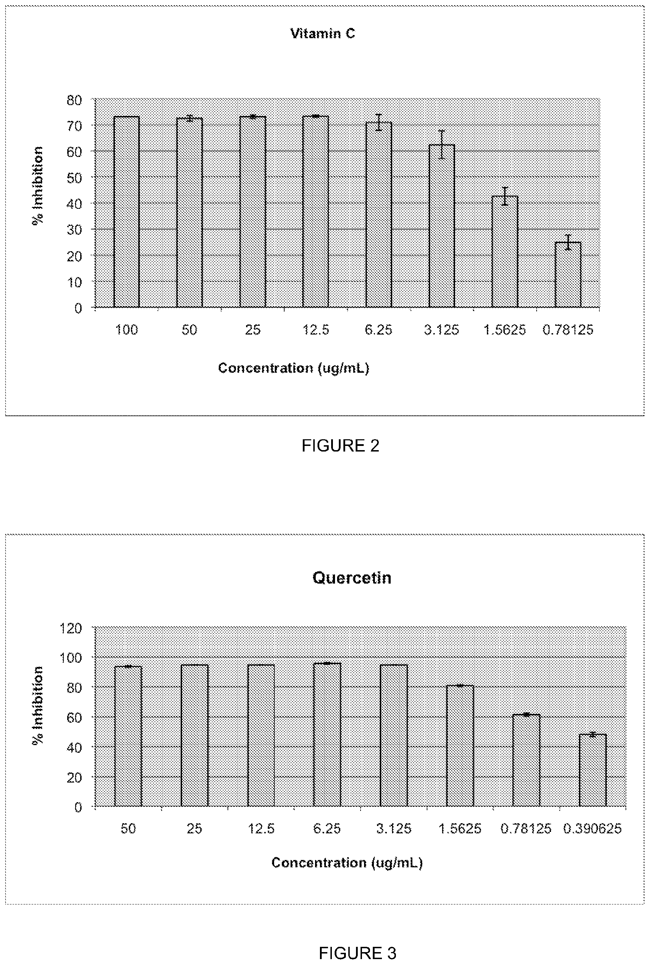

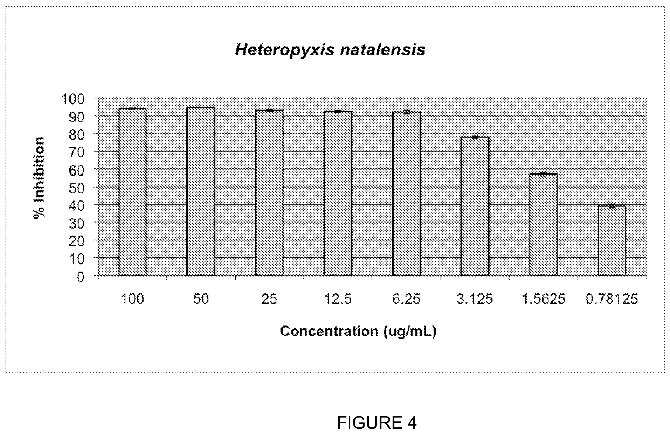

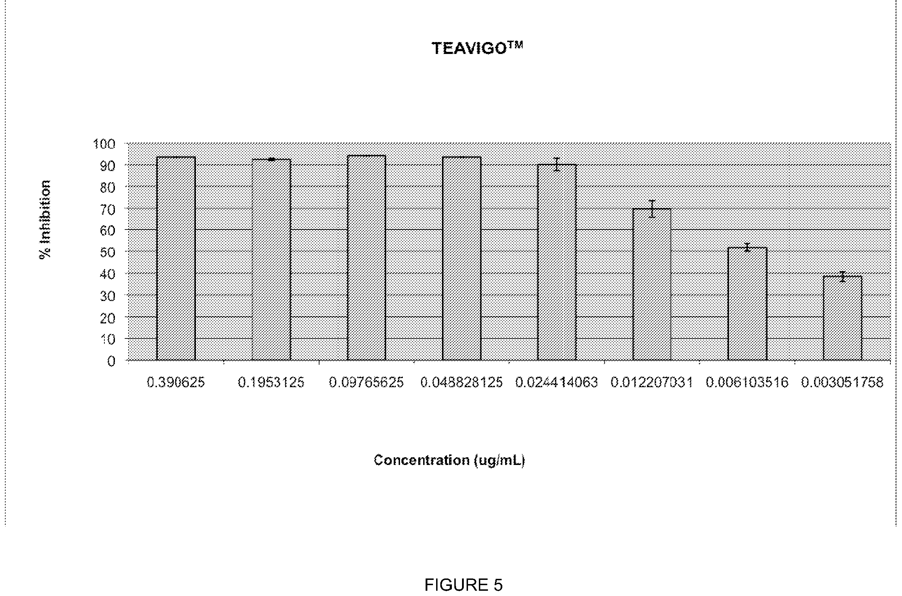

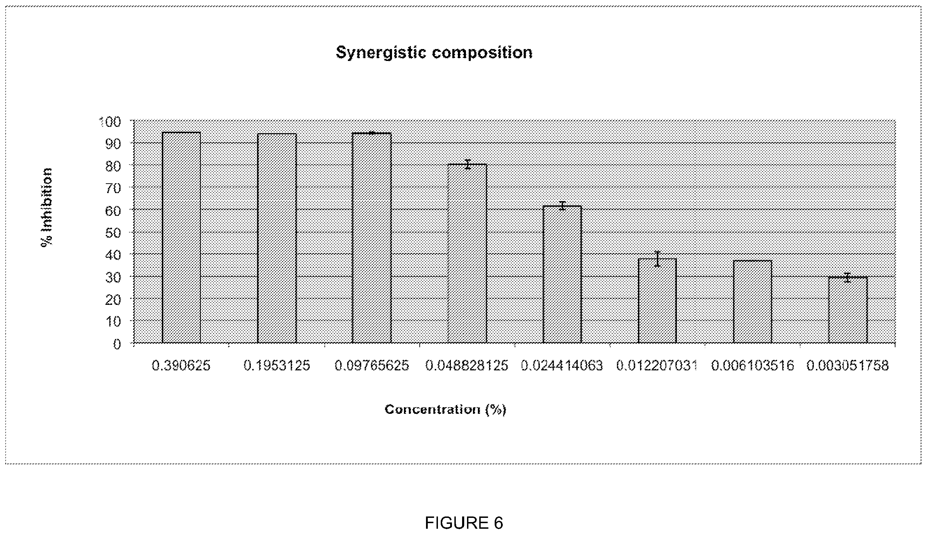

The percentage inhibition of DPPH, where DPPH turned colourless, was graphically determined as shown in Figures 2 to 6.

In a study conducted by Muchuweti *et al.* (2006), a 70% ethanol extract of the leaves and twigs of *H. natalensis* at a concentration of 32.26 µg/ml had a 29.65 inhibition percentage. A much higher inhibition percentage (>90 %) is obtained in this investigation at the same concentration.

*Melaleuca alternifolia* and *M. piperita* did not exhibit inhibition of DPPH at the highest concentration tested (0.1% v/v). The IC₅₀ is 50% of DPPH that is inhibited / turned colourless by the samples tested and was calculated using GraphPad Prism 4 (San Diego, CA, USA).

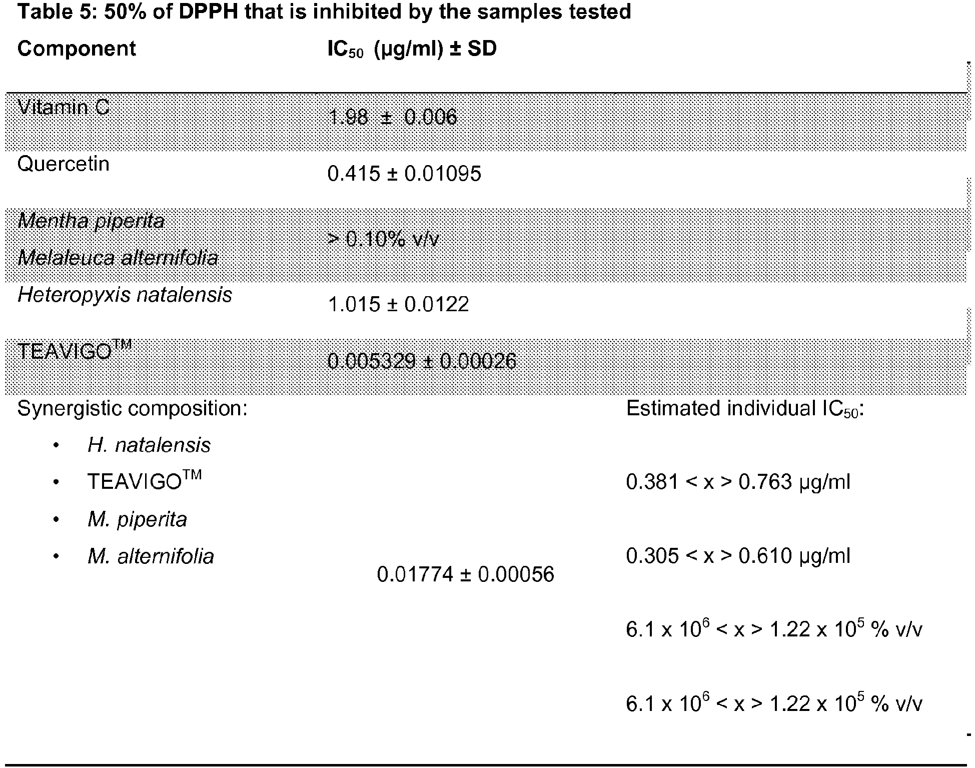

*Heteropyxis natalensis,* Quercetin and TEAVIGO™ all exhibit lower IC₅₀ values than the positive control vitamin C (1.98 ± 0.006 µg/ml). According to du Toit *et al.* (2001), greater antioxidant and free radical savaging activities are exhibited by flavonoids than vitamins C and E on an equimolar basis. As Quercetin is a flavonoid and *H. natalensis* has been shown to contain several other flavonoids, this may explain why these two samples show better antioxidant activity than vitamin C. The IC₅₀ value of the synergistic composition is given as 100% representing the highest values tested in combination, namely *H. natalensis* at 3.125 mg/ml, *M. alternifolia* and *M. piperita* at 0.05% and TEAVIGO™ at 2.5 mg/ml. The estimated IC₅₀ values are also given for each individual component of the synergistic combination.

### Conclusion

*Heteropyxis natalensis* exhibited activity against the Gram-positive microorganisms, *A. israelii* and *S*. *mutans* and the Gram-negative bacteria, *P. intermedia. Heteropyxis natalensis exhibited* moderate cytotoxicity. The cytokine, IL-8, levels were not reduced when the extract of *H. natalensis* was utilized to prevent the interaction of *A. israelii* with the epithelial cells, HEp-2. *Heteropyxis natalensis* interferes with pellicle formation and glucan binding of *S*. *mutans* to the enamel surface of the tooth. Five previously isolated compounds were identified for the first time from the ethanolic extract of *H. natalensis* leaves and twigs. The compounds were identified as Aurentiacin A (**1**), Cardamomin (**2**), 5-hydroxy-7-methoxy-6-methylflavanone (**3**), Quercetin (**4**) and 3,5,7-trihydroxyflavan (**5**). The MICs of the compounds **1** and **4** were found to be 0.0625 mg/ml and 1 mg/ml respectively against *A. israelii.* Compounds **2** and **5** exhibited no activity under 1 mg/ml against *A. israelii.* From the antioxidant assay it was shown that *Heteropyxis natalensis* has exhibited remarkable antioxidant activity on its own and appears to have even better activity in the synergistic composition. This plant extract may therefore aid in preventing periodontal diseases due to an imbalance between oxidants and antioxidants. The synergistic assay showed that there is an overall increase in inhibitory activity when *H. natalensis* extract is used in combination with *Melaleuca alternifolia* essential oil, *Mentha piperita* essential oil, and concentrated green tea extract.

## Claims

1. An oral care composition for inhibiting the growth of potentially pathogenic oral microorganisms, said composition comprising an extract of *Heteropyxis* natalensis prepared by drying collected aerial plant material, comprising leaves and twigs of *H.natalensis,* grinding the plant material into a powder, extracting by mixing the powder with ethanol to produce an extraction, filtering the extraction and evaporating the ethanol to retain the extract;
the composition being formulated in an oral delivery system including any one of the group selected from capsules, tablets, mouth wash, gel, paste, toothpaste, impregnated dental floss and chewing gum.

2. The oral care composition as claimed in claim 1, in which the extract includes or is enriched for any one or more of the compounds selected from Aurentiacin A, Cardamomin, 5-hydroxy-7-methoxy-6-methylflavanone, Quercetin, 3,5,7-trihydroxyflavan or derivatives of these compounds.

3. The oral care composition as claimed in claim 1, in which the composition includes the extract of *Heteropyxis natalensis* and any one or both of at least one essential oil and at least one other plant extract.

4. The oral care composition as claimed in claim 3, in which the at least one essential oil includes any one or more selected from the group of *Melaleuca alternifolia* and *Mentha piperita,* and the at least one other plant extracts is in the form of green tea extract.

5. The oral care composition as claimed in claim 3, in which the composition includes *Melaleuca alternifolia* essential oil, *Mentha piperita* essential oil, and concentrated green tea extract.

6. The oral care composition as claimed in claim 5, in which the potentially pathogenic oral microorganism is any one of:
- *Prevotella intermedia,* in which the concentration of the *Heteropyxis natalensis* extract is at least 3.13 mg/ml, the concentration of the concentrated green tea extract is at least 2 mg/ml, the concentration of *Mentha piperita* essential oil is at least 0.05 % (v/v), and the concentration of *Melaleuca alternifolia* essential oil is at least 0.05% (v/v);
- *Candida albicans,* in which the concentration of the *Heteropyxis natalensis* extract is at least 3.13 mg/ml, the concentration of the concentrated green tea extract is at least 4 mg/ml, the concentration of *Mentha piperita* essential oil is at least 0.05 % (v/v), and the concentration of *Melaleuca alternifolia* essential oil is at least 0.01% (v/v); and
- *Streptococcus mutans,* in which the concentration of the *Heteropyxis natalensis* extract is at least 0.78 mg/ml, the concentration of the concentrated green tea extract is at least 0.78 mg/ml, the concentration of *Mentha piperita* essential oil is at least 0.002% (v/v), and the concentration of *Melaleuca alternifolia* essential oil is at least 0.0004% (v/v).

7. The oral care composition as claimed in claims 5, in which the potentially pathogenic oral microorganisms includes *Streptococcus mutans, Prevotella intermedia* and *Candida albicans* and the concentration of the *Heteropyxis natalensis* extract is at least 3.13 mg/ml, the concentration of the concentrated green tea extract is at least 4 mg/ml, the concentration of *Mentha piperita* essential oil is at least 0.05 % (v/v), and the concentration of *Melaleuca alternifolia* essential oil is at least 0.05% (v/v).

8. A substance or composition comprising the oral care composition as claimed in any of claims 1 to 7, for use in a method of treating periodontal disease.

9. An oral care composition for inhibiting the growth of potentially pathogenic oral microorganisms, said composition including Aurentiacin A or derivatives thereof.

10. A substance or composition comprising Aurentiacin A or derivatives thereof, for use in a method of treating periodontal disease.

## Patentansprüche

1. Mundpflegezusammensetzung zum Hemmen des Wachstums von potenziell pathogenen oralen Mikroorganismen, wobei die Zusammensetzung
ein Extrakt von *Heteropyxis natalensis* umfasst, zubereitet durch Trocknen von gesammeltem oberirdischem Pflanzenmaterial, umfassend Blätter und Zweige von *H.natalensis,* Mahlen des Pflanzenmaterials zu einem Pulver, Extrahieren durch Mischen des Puders mit Ethanol, um ein Extrakt zu erzeugen, Filtern des Extraktes und Verdampfen des Ethanols, um das Extrakt zu erhalten;
wobei die Zusammensetzung in einem oralen Verabreichungssystem formuliert ist, umfassend eines aus der Gruppe ausgewählt aus Kapseln, Tabletten, Mundwasser, Gel, Paste, Zahnpasta, imprägnierte Zahnseide und Kaugummi.

2. Mundpflegezusammensetzung nach Anspruch 1, bei der das Extrakt enthält oder angereichert ist mit einem oder mehreren der Verbindungen, ausgewählt aus Aurentiacin A, Cardamomin, 5-Hydroxy-7-Methoxy-6-Methylflavon, Quercetin, 3,5,7-Trihydroxyflavan oder Derivate dieser Verbindungen.

3. Mundpflegezusammensetzung nach Anspruch 1, bei der die Zusammensetzung den Extrakt von *Heteropyxis natalensis* und irgendeine oder beide von zumindest einem ätherischen Öl und zumindest einem weiteren Pflanzenextrakt enthält.

4. Mundpflegezusammensetzung nach Anspruch 3, bei der das zumindest eine ätherische Öl eines oder mehreres, ausgewählt aus der Gruppe von *Melaleuca alternifolia* und *Mentha Piperita,* enthält und das zumindest eine weitere Pflanzenextrakt in Form von Extrakt von grünem Tee vorliegt.

5. Mundpflegezusammensetzung nach Anspruch 3, bei der die Zusammensetzung ätherisches Öl *Melaleuca alternifolia,* ätherisches Öl *Mentha Piperita* und konzentrierten Extrakt von grünem Tee enthält.

6. Mundpflegezusammensetzung nach Anspruch 5, bei der der potenziell pathogene orale Mikroorganismus eines ist von:
- *Prevotella intermedia,* bei dem die Konzentration des Extraktes von *Heteropyxis natalensis* mindestens 3,13 mg/ml, die Konzentration des konzentrierten Extraktes von grünem Tee mindestens 2 mg/ml, die Konzentration des ätherischen Öls *Mentha* Piperita mindestens 0,05 % (v/v) und die Konzentration des ätherischen Öls *Melaleuca alternifolia* mindestens 0,05 % (v/v) beträgt;
- *Candida albicans,* bei dem die Konzentration des Extraktes von *Heteropyxis natalensis* mindestens 3,13 mg/ml, die Konzentration des konzentrierten Extraktes von grünem Tee mindestens 4 mg/ml, die Konzentration des ätherischen Öls *Mentha Piperita* mindestens 0,05 % (v/v) und die Konzentration des ätherischen Öls *Melaleuca alternifolia* mindestens 0,01 % (v/v) beträgt; und
- *Streptococcus mutans,* bei dem die Konzentration des Extraktes von *Heteropyxis natalensis* mindestens 0,78 mg/ml, die Konzentration des konzentrierten Extraktes von grünem Tee mindestens 0,78 mg/ml, die Konzentration des ätherischen Öls *Mentha Piperita* mindestens 0,002 % (v/v) und die Konzentration des ätherischen Öls *Melaleuca alternifolia* mindestens 0,0004 % (v/v) beträgt.

7. Mundpflegezusammensetzung nach Anspruch 5, bei der die potenziell pathogenen oralen Mikroorganismen *Streptococcus mutans, Prevotella intermedia* und *Candida albicans* umfassen und die Konzentration des Extraktes von *Heteropyxis natalensis* mindestens 3,13 mg/ml, die Konzentration des konzentrierten Extraktes von grünem Tee mindestens 4 mg/ml, die Konzentration des ätherischen Öls *Mentha Piperita* mindestens 0,05 % (v/v) und die Konzentration des ätherischen Öls *Melaleuca alternifolia* mindesten 0,05 % (v/v) beträgt.

8. Substanz oder Zusammensetzung, umfassend die Mundpflegezusammensetzung nach einem der Ansprüche 1 bis 7, zur Verwendung in einem Verfahren zum Behandeln von Parodontitis.

9. Mundpflegezusammensetzung zum Hemmen des Wachstums von potenziell pathogenen oralen Mikroorganismen, wobei die Zusammensetzung Aurentiacin A oder Derivate davon enthält.

10. Substanz oder Zusammensetzung, umfassend Aurentiacin A oder Derivate davon, zur Verwendung in einem Verfahren zum Behandeln von Parodontitis.

## Revendications

1. Composition de soins bucco-dentaires pour inhiber la croissance de micro-organismes oraux potentiellement pathogènes, ladite composition comprenant
un extrait d'*Heteropyxis natalensis* préparé en séchant du matériel végétal aérien comprenant des feuilles et des brindilles de *H.natalensis,* en broyant le matériel végétal en poudre, en extrayant un produit d'extraction par mélange de la poudre avec de l'éthanol, en filtrant l'extraction et en évaporant le solvant pour retenir l'extrait;
la composition étant formulée dans un système d'administration comprenant l'un quelconque du groupe choisi parmi les capsules, les comprimés, le bain de bouche, le gel, la pâte, le dentifrice, le fil dentaire imprégné et le chewing-gum.

2. Composition de soins bucco-dentaires selon la revendication 1, dans laquelle l'extrait comprend ou est enrichi pour un ou plusieurs des composés choisis parmi l'Aurentiacine A, la Cardamomin, la 5-hydroxy-7-méthoxy-6-méthylflavanone, la Quercétine, 3,5,7-trihydroxyflavan ou des dérivés de ces composés.

3. Composition de soins bucco-dentaires selon la revendication 1, dans laquelle la composition comprend l'extrait *d'Heteropyxis natalensis* et l'un quelconque ou les deux d'au moins une huile essentielle et d'au moins un autre extrait de plante.

4. Composition de soins bucco-dentaires selon la revendication 3, dans laquelle la au moins une huile essentielle comprend une ou plusieurs huile essentielles sélectionnées dans le groupe de *Melaleuca alternifolia* et *Mentha piperita,* et le au moins un autre extrait de plante est sous la forme d'un extrait de thé vert.

5. Composition de soins bucco-dentaires selon la revendication 3, dans laquelle la composition comprend de l'huile essentielle de *Melaleuca alternifolia,* de l'huile essentielle de *Mentha piperita* et de l'extrait concentré de thé vert.

6. Composition de soins bucco-dentaires selon la revendication 5, dans laquelle le micro-organisme oral potentiellement pathogène est l'un quelconque parmi :
- Prevotella intermedia, dans laquelle la concentration de l'extrait *d'Heteropyxis natalensis* est d'au moins 3,13 mg/ml, la concentration de l'extrait de thé vert concentré est d'au moins 2 mg/ml, la concentration d'huile essentielle de *Mentha piperita* est d'au moins 0,05% (v/v), et la concentration d'huile essentielle de *Melaleuca alternifolia* est d'au moins 0,05% (v/v);
- *Candida albicans,* dans laquelle la concentration de l'extrait *d'Heteropyxis natalensis* est d'au moins 3,13 mg/ml, la concentration de l'extrait de thé vert concentré est d'au moins 4 mg/ml, la concentration d'huile essentielle de *Mentha piperita* est d'au moins 0,05% (v/v), et la concentration d'huile essentielle de *Melaleuca alternifolia* est d'au moins 0,01% (v/v); et
- *Streptococcus mutans,* dans lequel la concentration de l'extrait *d'Heteropyxis natalensis* est d'au moins 0,78 mg/ml, la concentration de l'extrait de thé vert concentré est d'au moins 0,78 mg/ml, la concentration d'huile essentielle de *Mentha piperita* est d'au moins 0,002% (v/v), et la concentration d'huile essentielle de *Melaleuca alternifolia* est d'au moins 0,0004% (v/v).

7. Composition de soins bucco-dentaires selon la revendication 5, dans laquelle les micro-organismes oraux potentiellement pathogènes comprennent *Streptococcus mutans, Prevotella intermedia* et *Candida albicans* et la concentration de l'extrait d'*Heteropyxis natalensis* est d'au moins 3,13 mg/ml, la concentration de l'extrait de thé vert concentré est d'au moins 4 mg/ml, la concentration d'huile essentielle de *Mentha piperita* est d'au moins 0,05% (v/v), et la concentration d'huile essentielle de *Melaleuca alternifolia* est d'au moins 0,05% (v/v).

8. Substance ou composition comprenant la composition de soins bucco-dentaires selon l'une quelconque des revendications 1 à 7, pour une utilisation dans une méthode de traitement de la parodontite.

9. Composition de soins bucco-dentaires pour inhiber la croissance de micro-organismes oraux potentiellement pathogènes, ladite composition comprenant de l'Aurentiacine A ou des dérivés de celle-ci.

10. Substance ou composition comprenant de l'Aurentiacine A ou des dérivés de celle-ci, pour une utilisation dans une méthode de traitement de la parodontite.
